# EUROPEAN PATENT APPLICATION

(11) **EP 3 653 055 A1**
(43) Date of publication of application: **20.05.2020**
(21) Application number: 19202820.7
(22) Date of filing: 06.09.2002
(51) Int. Cl.: A01N 63/00, A61K 45/00, C12N 5/00, C12N 5/02, C12N 5/0784, C07K 14/54, A61K 39/00

(54) **COMPOSITIONS AND METHODS FOR PRIMING MONOCYTIC DENDRITIC CELLS AND T CELLS FOR TH1 RESPONSE**

(30) Priority: 06.09.2001 US 317592 P
(62) Divisional of application: 10181985.2
(71) Applicant: NorthWest Biotherapeutics, Inc., Bethesda, MD 20814 (US)
(72) Inventor: Bosch, Marnix, L., Medina, WA 98039 (US)
(74) Representative: Donald, Jenny Susan

(57) **Abstract**

The present invention provides compositions and methods for inducing maturation of immature dendritic cells (DC) and for priming those cells for inducing a type 1 immune response. The present invention also provides dendritic cell populations useful for activating and for preparing T cells polarized towards production of type 1 cytokines and/or a type 1 response. Similarly, activated, polarized T cell populations, and methods of making the same are provided.

## Description

### BACKGROUND OF THE INVENTION

Antigen presenting cells (APC) are important in eliciting an effective immune response. They not only present antigens to T cells with antigen-specific T cell receptors, but also provide the signals necessary for T cell activation. These signals remain incompletely defined, but involve a variety of cell surface molecules as well as cytokines or growth factors. The factors necessary for the activation of naive or unpolarized T cells may be different from those required for the re-activation of memory T cells. The ability of APC to both present antigens and deliver signals for T cell activation is commonly referred to as an accessory cell function. Although monocytes and B cells have been shown to be competent APC, their antigen presenting capacities *in vitro* appear to be limited to the re-activation of previously sensitized T cells, Hence, they are not capable of directly activating functionally naive or unprimed T cell populations.

Dendritic cells (DCs) are the professional antigen presenting cells of the immune system that are believed to be capable of activating both naive and memory T cells. Dendritic cells are increasingly prepared *ex vivo* for use in immunotherapy, particularly the immunotherapy of cancer. The preparation of dendritic cells with optimal immunostimulatory properties requires an understanding and exploitation of the biology of these cells for *ex vivo* culture Various protocols for the culture of these cells have been described, with various advantages ascribed to each protocol. Recent protocols include the use of serum-free media, and the employment of maturation conditions that impart the desired immunostimulatory properties to the cultured cells.

Maturation of dendritic cells is the process that converts immature DCs, which are phenotypically similar to skin Langerhans cells, to mature, antigen presenting cells that can migrate to the lymph nodes This process results in the loss of the powerful antigen uptake capacity that characterizes the immature dendritic cells, and in the up-regulation of expression of co-stimulatory cell surface molecules and various cytokines. Known maturation protocols are based on the *in vivo* environment that DCs are believed to encounter during or after exposure to antigens. The best example of this approach is the use of monocyte conditioned media (MCM) as a cell culture medium MCM is generated *in vitro* by culturing monocytes and used as a source of maturation factors The major components in MCM responsible for maturation are reported to be the (pro)inflammatory cytokines Interleukin 1 beta (IL-1β), Interleukin 6 (IL-6) and tumor necrosis factor alpha (TNFα). Other maturation factors include prostaglandin E2 (PGE2), poly-dldC, vasointestinal peptide (VIP), bacterial lipopolysaccharide (LPS), as well as mycobacteria or components of mycobacteria, such as specific cell wall constituents

Fully mature dendritic cells differ qualitatively and quantitatively from immature DCs. Fully mature DCs express higher levels of MHC class I and class II antigens, and of T cell costimulatory molecules, *ie,* CD80 and CD86. These changes increase the capacity of the dendritic cells to activate T cells because they increase antigen density on the cell surface, as well as the magnitude of the T cell activation signal through the counterparts of the costimulatory molecules on the T cells, *e.g,* like CD28 In addition, mature DC produce large amounts of cytokines, which stimulate and direct the T cell response. Two of these cytokines are Interleukin 10 (IL-10) and Interleukin (IL-12). These cytokines have opposing effects on the direction of the induced T cell response. IL-10 production results in the induction of a Th-2 type response, while IL-12 production results in a Th-1 type response. The latter response is particularly desirable where a cellular immune response is desired, such as, for example, in cancer immunotherapy. A Th-1 type response results in the induction and differentiation of cytotoxic T lymphocytes (CTL), which are the effector arm of the cellular immune system. This effector arm is most effective in combating tumor growth. IL-12 also induces growth of natural killer (NK) cells, and has anti-angiogenic activity, both of which are effective anti-tumor weapons. Thus, the use of dendritic cells that produce IL-12 are in theory optimally suited for use in immunostimulation,

Certain dendritic cell maturation agents, such as, for example, bacterial lipopolysaccharide, bacterial CpG DNA, double stranded RNA and CD40 ligand, have been reported to induce immature DC to produce IL-12 and to prime immature DC for a Th-1 response. In contrast, anti-inflammatory molecules such as IL-10, TGF-β, PGE-2 and corticosteriods inhibit IL-12 production, and can prime cells for a Th-2 response.

Recently, enhancement of IL-12 production by dendritic cells has been reported by combining interferon gamma with certain dendritic cell maturation factors, such as bacterial lipopolysaccharide (LPS) and CD40. Both LPS and CD40 have a known capacity to induce small amounts of IL-12 during maturation, however. Thus, it is possible that the addition of IFNγ merely enhances that production, Interferon gamma signaling uses the Jak2-Stat1 pathway, which includes tyrosine phosphorylation of the tyrosine residue at position 701 of Stat1 prior to its migration to the nucleus and the ensuing enhancement of transcription of interferon gamma-responsive genes. Very little is known, however, about signal transduction pathways in human monocyte-derived dendritic cells, The mechanism for interferon gamma action in these cells has not been established

The attenuated bovine strain (*Mycobacterium bovis*) of *Mycobacterium tuberculosis,* now known as bacille Calmette-Gueiin (BCG), has been used in cancer immunotherapy. In one example, intravesical administration of live BCG has proven effective for the treatment of bladder cancer, although the mechanism for this treatment is not known The effects of BCG administration are postulated to be mediated by the induction of an immune response that attacks, for example, cancer cells. The specific role of BCG in this response is thought to be that of a generalized inducer of immune reactivity, as well as having an adjuvant function in the presentation of tumor antigens to the immune system.

BCG has also been found to be a powerful maturation agent for dendritic cells, with the ability to up-regulate the maturation marker CD83. BCG can also up regulate MHC molecules and the costimulatory molecules CD80 and CD86, concomitant with a reduction in endocytic capacity, In addition, BCG, or BCG-derived lipoarabidomannans, has been reported to increase cytokine production, although contrary to results found with other DC maturation agents the production of IL-12 was found to be specifically inhibited. This latter property, the inhibition of IL-12 production, reduces the attraction of using BCG for the maturation of dendritic cells for immunotherapy in which a strong cell-mediated, cytotoxic response (Th-1 response), is desired

The use of BCG in active immunotherapy, thus, has the potential to induce dendritic cell maturation. There is a need, however, for compositions and methods of using such compositions that induce such maturation of dendritic cells and that simultaneously provide broad immune stimulation, and that prime those dendritic cells towards a type 1 (Th-1) immune response with a strong cytotoxic T cell response.

### BRIEF SUMMARY OF THE INVENTION

The present invention provides methods and compositions for inducing maturation of immature dendritic cells (DC), with an agent that simultaneously provide broad immune stimulation (*i*.*e.* BCG), and for priming those cells for an antigen-specific cytotoxic T cell response. In one aspect, a method is provided for producing a mature dendritic cell population, including providing immature dendritic cells; and contacting the immature dendritic cells with an effective concentration of BCG and Interferon gamma (IFNγ) under culture conditions suitable for maturation of the immature dendritic cells to form a mature dendritic cell population The mature dendritic cell population produces an increased ratio of Interleukin 12 to Interleukin 10 than an immature dendritic cell population not contacted with BCG and IFNγ alone during maturation. The immature dendritic cells can be contacted with a predetermined antigen prior to or during contacting with BCG and IFNγ. The predetermined antigen can be, for example, a tumor specific antigen, a tumor associated antigen, a viral antigen, a bacterial antigen, tumor cells, bacterial cells, recombinant cells expressing an antigen, a cell lysate, a membrane preparation, a recombinantly produced antigen, a peptide antigen (*e.g*., a synthetic peptide antigen), or an isolated antigen.

In certain embodiments, the method can optionally further include isolating monocytic dendritic cell precursors; and culturing the precursors in the presence of a differentiating agent to form a population immature dendritic cells. Suitable differentiating agents include, for example, GM-CSF, Interleukin 4, a combination of GM-CSF and Interleukin 4, or Interleukin 13. The monocytic dendritic cell precursors can be isolated from a human subject. In a particular embodiment, the mature dendritic cells produce a ratio of IL-12 to IL-10 of at least 1:1.

In another aspect, a method for producing a mature dendritic cell population is provided. The method generally includes providing immature dendritic cells; and contacting the immature dendritic cells with an effective amount of BCG and Interferon gamma (IFNγ) under culture conditions suitable for maturation of the immature dendritic cells to form a mature dendritic cell population The resulting mature dendritic cell population produces a type 1 immune response. The immature dendritic cells can be contacted with a predetermined antigen prior to or during contacting with BCG and IFNγ The predetermined antigen can be, for example, a tumor specific antigen, a tumor associated antigen, a viral antigen, a bacterial antigen, tumor cells, bacterial cells, recombinant cells expressing an antigen, a cell lysate, a membrane preparation, a recombinantly produced antigen, a peptide antigen (*i*.*e.*, a synthetic peptide), or an isolated antigen.

In certain embodiments, the method can optionally further include isolating monocytic dendritic cell precursors; and culturing the precursors in the presence of a differentiating agent to form the immature dendritic cells. Suitable differentiating agents include, for example, GM-CSF, Interleukin 4, a combination of GM-CSF and Interleukin 4, or Interleukin 13. The monocytic dendritic cell precursors are isolated from a human subject. In a particular embodiment, the mature dendritic cells produce a ratio of IL-12 to IL-10 of at least about 1:1.

In still another aspect, compositions for activating T cells are provided The compositions can include a dendritic cell populations matured with an effective concentration of BCG and IFNγ under suitable conditions for maturation; and a predetermined antigen. The dendritic cell population can produce an increased ratio of Interleukin 12 (IL-12) to Interleukin 10 (IL-10) than a mature dendritic cell population contacted with BCG without IFNγ during maturation In certain embodiments, the dendritic cell population can produce IL-12 to IL-10 in a ratio of at least about 10:1. In other embodiments, the dendritic cell population can produce IL-12 to IL-10 in a ratio of at least about 100:1 than a similar immature dendritic cell population cultured in the presence of BCG without IFNγ during maturation.

In another aspect, an isolated, immature dendritic cell population is provided. The cell population includes immature monocytic dendritic cells, and an effective concentration of BCG and IFNγ to induce maturation of the immature dendritic cells. The resulting mature dendritic cells produce more Interleukin 12 (IL-12) to Interleukin 10 (IL-10) than a similar immature dendritic cell population cultured in the presence of BCG without IFNγ during maturation. The cell population can optionally include a predetermined antigen and/or isolated T cells, such as naive T cells. The T cell can optionally be present in a preparation of isolated lymphocytes.

A method for producing activated T cells is also provided. The method generally includes providing immature dendritic cells; contacting the immature dendritic cells with a predetermined antigen; and contacting the immature dendritic cells with an effective concentration of BCG and IFNγ under culture conditions suitable for maturation of the immature dendritic cells to form mature dendritic cells. The mature dendritic cells can be contacted with naive T cells to form activated T cells producing IFNγ and/or polarized for a type 1 (Th-1) response. Suitable antigens include, for example, a tumor specific antigen, a tumor associated antigen, a viral antigen, a bacterial antigen, tumor cells, bacterial cells, recombinant cells expressing an antigen, a cell lysate, a membrane preparation, a recombinantly produced antigen, a peptide antigen (*e.g*., a synthetic peptide antigen), or an isolated antigen.

The immature dendritic cells can be contacted simultaneously with the predetermined antigen, BCG and IFNγ. or the cells can be contacted with the predetermined antigen prior to contacting with BCG and IFNγ. In certain embodiments, the method can further include isolating monocytic dendritic cell precursors; and culturing the precursors in the presence of a differentiation agent to induce the formation of the immature dendritic cells. Suitable differentiating agents include, for example, GM-CSF, Interleukin 4, a combination of GM-CSF and Interleukin 4, or Interleukin 13. The monocytic dendritic cell precursors can optionally be isolated from a human subject. In a particular embodiment, the immature dendritic cells and T cells are autologous to each other.

Isolated mature dendritic cells producing more Interleukin 12 (IL-12) to Interleukin 10 (IL-10) are also provided. The mature dendritic cells can be provided by maturation of immature dendritic cells with a composition comprising effective concentrations of BCG and IFNγ under conditions suitable for the maturation of the dendritic cells. A predetermined antigen can optionally be included with the isolated, mature dendritic cells. Isolated mature dendritic cells loaded with a predetermined antigen are also provided. The dendritic cells can produce more Interleukin 12 (IL-12) than Interleukin 10 (IL-10), such as, for example, at least 10-fold more IL-12 than IL-10 than a similar immature dendritic cell population cultured in the presence of BCG without IFNγ during maturation.

A method for producing a type 1 (Th-1) immune response in an animal is also provided. The method generally includes providing immature dendritic cells; contacting the immature dendritic cells with effective amounts of BCG and Interferon gamma (IFNγ), and a predetermined antigen under culture conditions suitable for maturation of the immature dendritic cells to form mature dendritic cells. The mature dendritic cells can either be administered to an animal or can be contacted with naïve T cells to form activated T cells characterized by the production of Interferon gamma (IFNγ) and/or tumor necrosis factor α (TNFα). The activated T cells can be administered to the animal in need of stimulation of a cytotoxic T cell response to the specific antigen Suitable antigens include, for example, a tumor specific antigen, a tumor associated antigen, a viral antigen, a bacterial antigen, tumor cells, bacterial cells, recombinant cells expressing an antigen, a cell lysate, a membrane preparation, a recombinantly produced antigen, a peptide antigen (*e.g.*, a synthetic peptide antigen), or an isolated antigen. The immature dendritic cells can optionally be simultaneously contacted with the predetermined antigen, BCG and IFNγ. or the immature dendritic cells can be contacted with the predetermined antigen prior to contacting with BCG and IFNγ.

In certain embodiments, the method can further include isolating monocytic dendritic cell precursors from the animal; and culturing the precursors in the presence of a differentiating agent to form the immature dendritic cells. The differentiating agent can be, for example, GM-CSF, Interleukin 4, a combination of GM-CSF and Interleukin 4, or Interleukin 13.

The immature dendritic cells and T cells can be autologous to the animal, or allogenic to the animal. Alternatively, the immature dendritic cells and T cells can have the same MHC haplotype as the animal, or share an MHC marker In certain embodiments, the animal can be human, or can be a non-human animal.

### DETAILED DESCRIPTION OF THE INVENTION

The present invention provides methods for inducing maturation of immature dendritic cells (DC) and for priming those cells for an antigen-specific cytotoxic T cell response (Th-1 response). The present invention also provides dendritic cell populations useful for activating and for preparing T cell populations polarized towards production of type 1 cytokines (*e.g.*, IFNγ, TNFα, and/or IL-2). Such dendritic cell populations include immature monocytic dendritic cells contacted with BCG, IFNγ and a predetermined antigen under suitable maturation conditions. The immature dendritic cells can be contacted with the antigen either during or prior to maturation Alternatively, immature monocytic dendritic cells, already exposed to antigen (*e.g., in vivo*)*,* can be contacted with BCG and IFNγ under suitable maturation conditions. The resulting mature dendritic cells are primed to activate and polarize T cells towards a type 1 response. A type 1 response includes production of type 1 cytokines (*e.g.*, IFNγ. and/or IL-2), production of more IL-12 than IL-10, a cytotoxic T cell response, production of Th-1 cells, and production of certain types of antibodies. Tumor Necrosis Factor α (TNFα) can also be upregulated. In contrast, a type 2 response is characterized by production of IL-4, IL-5 and IL-10, production of more IL-10 than IL-12, production of Th2 cells, and lack of induction of a CTL response.

In a related aspect, compositions are provided comprising a maturation agent for immature dendritic cells, such as monocytic dendritic cell precursors, which can also prime those dendritic cells for a type 1 response. Such mature, primed monocytic dendritic cells can increase Major Histocompatibility Complex (MHC) class-I presentation of a predetermined antigen, *i.e*., a predetermined exogenous antigen. MHC class I presentation of antigen is desired to induce differentiation of cytotoxic T lymphocytes (CTL) and stimulation of antigen-specific CTL-mediated lysis of target cells. Such compositions include BCG and IFNγ which can be admixed with a cell population comprising immature dendritic cells, to mature the immature dendritic cells, and to convert or overcome the inhibition of IL-12 induced by contact of the immature dendritic cells with BCG. Immature dendritic cells contacted with such compositions undergo maturation and typically produce greater amounts of IL-12 than IL-10, as compared with an immature dendritic cell population contacted with BCG alone.

In another aspect, monocytic dendritic cells precursors obtained from subjects or donors can be contacted with cytokines (*e.g.,* GM-CSF and IL-4) to obtain immature dendritic cells The immature dendritic cells can then be contacted with a predetermined antigen, either in combination with BCG and IFNγ alone, or in combination with a cytokine, to mature the dendritic cells and to prime the cells for inducing a type 1 immune response in T cells In certain embodiments, MHC Class-I antigen processing is stimulated, which is useful to elicit a CTL response against cells displaying the predetermined antigen

Dendritic cells are a diverse population of antigen presenting cells found in a variety of lymphoid and non-lymphoid tissues. (*See* Liu, Cell 106:259-62 (2001); Steinman, Ann. Rev. Immunol 9:271-96 (1991)) Dendritic cells include lymphoid dendritic cells of the spleen, Langerhans cells of the epidermis, and veiled cells in the blood circulation, Collectively, dendritic cells are classified as a group based on their morphology, high levels of surface MHC-class II expression, and absence of certain other surface markers expressed on T cells, B cells, monocytes, and natural killer cells. In particular, monocyte-derived dendritic cells (also referred to as monocytic dendritic cells) usually express CD11c, CD80, CD86, and are HLA-DR⁺, but are CD14⁺.

In contrast, monocytic dendritic cell precursors (typically monocytes) are usually CD14⁺. Monocytic dendritic cell precursors can be obtained from any tissue where they reside, particularly lymphoid tissues such as the spleen, bone marrow, lymph nodes and thymus Monocytic dendritic cell precursors also can be isolated from the circulatory system. Peripheral blood is a readily accessible source of monocytic dendritic cell precursors Umbilical cord blood is another source of monocytic dendritic cell precursors. Monocytic dendritic cell precursors can be isolated from a variety of organisms in which an immune response can be elicited Such organisms include animals, for example, including humans, and non-human animals, such as, primates, mammals (including dogs, cats, mice, and rats), birds (including chickens), as well as transgenic species thereof.

In certain embodiments, the monocytic dendritic cell precursors and/or immature dendritic cells can be isolated from a healthy subject or from a subject in need of immunostimulation, such as, for example, a prostate cancer patient or other subject for whom cellular immunostimulation can be beneficial or desired (*i.e.,* a subject having a bacterial or vital infection, and the like). Dendritic cell precursors and/or immature dendritic cells also can be obtained from an HLA-matched healthy individual for administration to an HLA-matched subject in need of immunostimulation.

### Dendritic Cell Precursors and Immature Dendritic Cells

Methods for isolating cell populations enriched for dendritic cell precusors and immature dendritic cells from various sources, including blood and bone marrow, are known in the art. For example, dendritic cell precursors and immature dendritic cells can be isolated by collecting heparinized blood, by apheresis or leukapheresis, by preparation of buffy coats, resetting, centrifugation, density gradient centrifugation (*e.g.,* using Ficoll (such as FICOLL-PAQUE®), PERCOLL® (colloidal silica particles (15-30 mm diameter) coated with non-dialyzable polyvinylpyrrolidone (PVP)), sucrose, and the like), differential lysis of cells, filtration, and the like. In certain embodiments, a leukocyte population can be prepared, such as, for example, by collecting blood from a subject, defribrinating to remove the platelets and lysing the red blood cells. Dendritic cell precursors and immature dendritic cells can optionally be enriched for monocytic dendritic cell precursors by, for example, centrifugation through a PERCOLL® gradient

Dendritic cell precursors and immature dendritic cells optionally can be prepared in a closed, aseptic system. As used herein, the terms "closed, aseptic system" or "closed system" refer to a system in which exposure to non-sterilize, ambient, or circulating air or other non-sterile conditions is minimized or eliminated. Closed systems for isolating dendritic cell precursors and immature dendritic cells generally exclude density gradient centrifugation in open top tubes, open air transfer of cells, culture of cells in tissue culture plates or unsealed flasks, and the like. In a typical embodiment, the closed system allows aseptic transfer of the dendritic cell precursors and immature dendritic cells from an initial collection vessel to a sealable tissue culture vessel without exposure to non-sterile air.

In certain embodiments, monocytic dendritic cell precursors are isolated by adherence to a monocyte-binding substrate, as disclosed in U.S. Patent Application No. 60/307,978, filed July 25, 2001 (Attorney Docket No 020093-002600US), the disclosure of which is incorporated by reference herein, For example, a population of leukocytes (*e.g*., isolated by leukapheresis) can be contacted with a monocytic dendritic cell precursor adhering substrate. When the population of leukocytes is contacted with the substrate, the monocytic dendritic cell precursors in the leukocyte population preferentially adhere to the substrate. Other leukocytes (including other potential dendritic cell precursors) exhibit reduced binding affinity to the substrate, thereby allowing the monocytic dendritic cell precursors to be preferentially emiched on the surface of the substrate.

Suitable substrates include, for example, those having a large surface area to volume ratio. Such substrates can be, for example, a particulate or fibrous substrate. Suitable particulate substrates include, for example, glass particles, plastic particles, glass-coated plastic particles, glass-coated polystyrene particles, and other beads suitable for protein absorption. Suitable fibrous substrates include microcapillary tubes and microvillous membrane. The particulate or fibrous substrate usually allows the adhered monocytic dendritic cell precursors to be eluted without substantially reducing the viability of the adhered cells. A particulate or fibrous substrate can be substantially non-porous to facilitate elution of monocytic dendritic cell precursors or dendritic cells from the substrate. A "substantially non-porous" substrate is a substrate in which at least a majority of pores present in the substrate are smaller than the cells to minimize entrapping cells in the substrate.

Adherence of the monocytic dendritic cell precursors to the substrate can optionally be enhanced by addition of binding media Suitable binding media include monocytic dendritic cell precursor culture media (*e.g*., AIM-V®, RPMI 1640, DMEM, X-VIVO 15®, and the like) supplemented, individually or in any combination, with for example, cytokines (*e.g.*, Granulocyte/Macrophage Colony Stimulating Factor (GM-CSF), Interleukin 4 (IL-4), or Interleukin 13 (IL-13)), blood plasma, serum (*e.g*., human serum, such as autologous or allogenic sera), purified proteins, such as serum albumin, divalent cations (*e.g.,* calcium and/or magnesium ions) and other molecules that aid in the specific adherence of monocytic dendritic cell precursors to the substrate, or that prevent adherence of non-monocytic dendritic cell precursors to the substrate. In certain embodiments, the blood plasma or serum can be heated-inactivated. The heat-inactivated plasma can be autologous or heterologous to the leukocytes.

Following adherence of monocytic dendritic cell precursors to the substrate, the non-adhering leukocytes are separated from the monocytic dendritic cell precursor/substrate complexes. Any suitable means can be used to separate the non-adhering cells from the complexes. For example, the mixture of the non-adhering leukocytes and the complexes can be allowed to settle, and the non-adhering leukocytes and media decanted or drained. Alternatively, the mixture can be centrifuged, and the supernatant containing the non-adhering leukocytes decanted or drained from the pelleted complexes.

Isolated dendritic cell precursors can be cultured *ex vivo* for differentiation, maturation and/or expansion (As used herein, isolated immature dendritic cells, dendritic cell precursors, T cells, and other cells, refers to cells that, by human hand, exists apart from their native environment, and are therefore not a product of nature. Isolated cells can exist in purified form, in semi-purified form, or in a non-native environment.) Briefly, *ex vivo* differentiation typically involves culturing dendritic cell precursors, or populations of cells having dendritic cell precursors, in the presence of one or more differentiation agents. Suitable differentiating agents can be, for example, cellular growth factors (*e*.*g*., cytokines such as (GM-CSF), Interleukin 4 (IL-4), Interleukin 13 (IL-13), and/or combinations thereof). In certain embodiments, the monocytic dendritic cells precursors are differentiated to form monocyte-derived immature dendritic cells

The dendritic cell precursors can be cultured and differentiated in suitable culture conditions Suitable tissue culture media include AIM-V®, RPMI 1640, DMEM, X-VTVO 15®, and the like. The tissue culture media can be supplemented with serum, amino acids, vitamins, cytokines, such as GM-CSF and/or IL-4, divalent cations, and the like, to promote differentiation of the cells. In certain embodiments, the dendritic cell precursors can be cultured in the serum-free media Such culture conditions can optionally exclude any animal-derived products. A typical cytokine combination in a typical dendritic cell culture medium is about 500 units/ml each of GM-CSF and IL-4. Dendritic cell precursors, when differentiated to form immature dendritic cells, are phenotypically similar to skin Langerhans cells. Immature dendritic cells typically are CD14⁻ and CD11c⁺, express low levels of CD86 and CD83, and are able to capture soluble antigens via specialized endocytosis.

The immature dendritic cells are matured to form mature dendritic cells. Mature DC lose the ability to take up antigen and display up-regulated expression of costimulatory cell surface molecules and various cytokines Specifically, mature DC express higher levels of MHC class I and II antigens than immature dendritic cells, and mature dendritic cells are generally identified as being CD80⁺, CD83⁺, CD86⁺, and CD14⁻. Greater MHC expression leads to an increase in antigen density on the DC surface, while up regulation of costimulatory molecules CD80 and CD86 strengthens the T cell activation signal through the counterparts of the costimulatory molecules, such as CD28 on the T cells.

Mature dendritic cells of the present invention can be prepared (*i*.e., matured) by contacting the immature dendritic cells with effective amounts or concentrations of BCG and IFNγ. Effective amounts of BCG typically range from about 10⁵ to 10⁷ cfu per milliliter of tissue culture media. Effective amounts of IFNγ typically range from about 100-1000 U per milliliter of tissue culture media. Bacillus Calmette-Guerin (BCG) is an avirulent strain of *M*. *bovis*. As used herein, BCG refers to whole BCG as well as cell wall constituents, BCG-derived lipoarabidomannans, and other BCG components that are associated with induction of a type 2 immune response. BCG is optionally inactivated, such as heat-inactivated BCG, formalin-treated BCG, and the like

BCG increases expression of the surface maturation markers CD83 and CD86 on dendritic cells, concomitant with inhibition of IL-12 production and the exclusion of antigens from endocytosis Without intending to be bound by any particular theory, dendritic cell maturation by BCG also has been characterized as involving homotypic aggregation and the release of tumor necrosis factor-α (TNFα). (*See, e.g.,* Thurnher, et al., Int. J. Cancer 70:128-34 (1997) incorporated herein by reference.) Maturing the immature dendritic cells with IFNγ and BCG promotes DC production of IL-12, and reduces or inhibits production of IL-10, thereby priming the mature dendritic cells for a type 1 (Th-1) response.

The immature DC are typically contacted with effective amounts of BCG and IFNγ for about one hour to about 24 hours The immature dendritic cells can be cultured and matured in suitable maturation culture conditions. Suitable tissue culture media include AIM-V®, RPMI 1640, DMEM, X-VIVO 15®, and the like. The tissue culture media can be supplemented with amino acids, vitamins, cytokines, such as GM-CSF and/or IL-4, divalent cations, and the like, to promote maturation of the cells. A typical cytokine combination is about 500 units/ml each of GM-CSF and IL-4.

Maturation of dendritic cells can be monitored by methods known in the art Cell surface markers can be detected in assays familiar to the art, such as flow cytometry, immunohistochemistry, and the like. The cells can also be monitored for cytokine production (*e.g.*, by ELISA, FACS, or other immune assay). In a DC population matured according to the present invention, IL-12 levels are higher than IL-10 levels, to promote a type 1 (Th-1) response. For example, the DCs can produce ratios of IL-12/IL-10 from greater than 1:1, to about 10:1 or about 100:1. Mature DCs also lose the ability to uptake antigen by pinocytosis, which can be analyzed by uptake assays familiar to one of ordinary skill in the art Dendritic cell precursors, immature dendritic cells, and mature dendritic cells, either primed or unprimed, with antigens can be cryopreserved for use at a later date Methods for cryopreservation are well-known in the art See, for example, U.S. Patent 5,788,963 incorporated herein by reference in its entirety.

### Antigens

The mature, primed dendritic cells according to the present invention can present antigen to T cells. Mature, primed dendritic cells can be formed by contacting immature dendritic cells with a predetermined antigen either prior to or during maturation. Alternatively, immature dendritic cells that have already been contacted with antigen (*e.g.*, *in vivo* prior to isolation) can be contacted with a composition comprising BCG and IFNγ to form mature dendritic cells primed for a type 1 (Th-1) response.

Suitable predetermined antigens can include any antigen for which T-cell activation is desired. Such antigens can include, for example, bacterial antigens, tumor specific or tumor associated antigens (*e.g*., whole cells, tumor cell lysate, isolated antigens from tumors, fusion proteins, liposomes, and the like), viral antigens, and any other antigen or fragment of an antigen, *e.g*., a peptide or polypeptide antigen. In certain embodiments, the antigen can be, for example, but not limited to, prostate specific membrane antigen (PSMA), prostatic acid phosphatase (PAP), or prostate specific antigen (PSA). (*See, e.g.,* Pepsidero et al., Cancer Res 40:2428-32 (1980); McCormack et al., Urology 45:729-44 (1995).) The antigen can also be a bacterial cell, bacterial lysate, membrane fragment from a cellular lysate, or any other source known in the art. The antigen can be expressed or produced recombinantly, or even chemically synthesized, The recombinant antigen can also be expressed on the surface of a host cell (*e.g*., bacteria, yeast, insect, vertebrate or mammalian cells), can be present in a lysate, or can be purified from the lysate.

Antigen can also be present in a sample from a subject. For example, a tissue sample from a hyperproliferative or other condition in a subject can be used as a source of antigen. Such a sample can be obtained, for example, by biopsy or by surgical resection, Such an antigen can be used as a lysate or as an isolated preparation Alternatively, a membrane preparation of cells of a subject (*e.g*., a cancer patient), or an established cell lines also can be used as an antigen or source of antigen.

In an exemplary embodiment, a prostate tumor cell lysate recovered from surgical specimens can be used as a source of antigen For example, a sample of a cancer patient's own tumor, obtained at biopsy or at surgical resection, can be used directly to present antigen to dendritic cells or to provide a cell lysate for antigen presentation. Alternatively, a membrane preparation of tumor cells of a cancer patient can be used, The tumor cell can be prostatic, lung, ovarian, colon, brain, melanoma, or any other type of tumor cell. Lysates and membrane preparation can be prepared from isolate tumor cells by methods known in the art

In another exemplary embodiment, purified or semi-purified prostate specific membrane antigen (PSMA, also known as PSM antigen), which specifically reacts with monoclonal antibody 7E11-C5, can be used as antigen, (*See generally* Horoszewicz et al., Prog. Clin Bioll.Res. 37:115-32 (1983), U.S. Patent No. 5,162,504; U.S. Patent No 5,788,963; Feng et al., Proc. Am. Assoc Cancer Res. 32:(Abs, 1418)238 (1991); the disclosures of which are incorporated by reference herein.) In yet another exemplary embodiment, an antigenic peptide having the amino acid residue sequence Leu Leu His Glu Thr Asp Ser Ala Val (SEQ ID NO:1) (designated PSM-P1), which corresponds to amino acid residues 4-12 of PSMA, can be used as an antigen. Alternatively, an antigenic peptide having the amino acid residue sequence Ala Leu Phe Asp Ile Glu Ser Lys Val (SEQ ID NO:2) (designated PSM-P2), which corresponds to amino acid residues 711-719 of PSMA, can be used as antigen.

In a particular embodiment, an antigenic peptide having an amino acid residue sequence Xaa Leu (or Met) Xaa Xaa Xaa Xaa Xaa Xaa Val (or Leu) (designated PSM-PX), where Xaa represents any amino acid residue, can be used as antigen. This peptide resembles the HLA-A0201 binding motif, *i.e*., a binding motif of 9-10 amino acid residues with "anchor residues", leucine and valine found in HLA-A2 patients. (*See, e.g.,* Grey et al., Cancer Surveys 22:37-49 (1995)) This peptide can be used as antigen for HLA-A2⁺ patients (see, Central Data Analysis Committee "Allele Frequencies", Section 6.3, Tsuji, K. et al. (eds.), Tokyo University Press, pp. 1066-1077) Similarly, peptides resembling other HLA binding motifs can be used.

Typically, immature dendritic cells are cultured in the presence of BCG, IFNγ and the predetermined antigen under suitable maturation conditions, as described above. Optionally, the immature dendritic cells can be admixed with the predetermined antigen in a typical dendritic cell culture media without GM-CSF and IL-4, or a maturation agent Following at least about 10 minutes to 2 days of culture with the antigen, the antigen can be removed and culture media supplemented with BCG and IFNγ can be added. Cytokines (*e.g*, GM-CSF and IL-4) can also be added to the maturation media. Methods for contacting dendritic cells with are generally known in the art (*See generally* Steel and Nutman, J. Immunol 160:351-60 (1998); Tao et al., J. Immunol 158:4237-44 (1997); Dozmorov and Miller, Cell Immunol. 178:187-96 (1997); Inaba et al., J Exp Med 166:182-94 (1987); Macatonia et al., J Exp Med 169:1255-64 (1989); De Bruijn et al., Eur. J. Immunol, 22:3013-20 (1992); the disclosures of which are incorporated by reference herein.)

The resulting mature, primed dendritic cells are then co-incubated with T cells, such as naive T cells T cells, or a subset of T cells, can be obtained from various lymphoid tissues for use as responder cells. Such tissues include but are not limited to spleen, lymph nodes, and/or peripheral blood. The cells can be co-cultured with mature, primed dendritic cells as a mixed T cell population or as a purified T cell subset. T cell purification can be achieved by positive, or negative selection, including but not limited to, the use of antibodies directed to CD2, CD3, CD4, CD8, and the like.

By contacting T cells with mature, primed dendritic cells, antigen-reactive, or activated, polarized T cells or T lymphocytes are provided. As used herein, the term "polarized" refers to T cells that produce high levels of IFNγ or are otherwise primed for inducing a type 1 (Th-1) response. Such methods typically include contacting immature dendritic cells with BCG and IFNγ to prepare mature, primed dendritic cells. The immature dendritic cells can be contacted with a predetermined antigen during or prior to maturation. The immature dendritic cells can be co-cultured with T cells (*e.g*., naive T cells) during maturation, or co-cultured with T cells (*e.g.*, naïve T cells) after maturation and priming of the dendritic cells for inducing a type 1 response. The immature dendritic cells or mature dendritic cells can be enriched prior to maturation In addition, T cells can be enriched from a population of lymphocytes prior to contacting with the dendritic cells. In a specific embodiment, enriched or purified populations of CD4⁺ T cells are contacted with the dendritic cells Co-culturing of mature, primed dendritic cells with T cells leads to the stimulation of specific T cells which mature into antigen-reactive CD4⁺ T cells or antigen-reactive CD8⁺ T cells.

In another aspect, methods are provided for re-stimulation of T cells in *vitro,* by culturing the cells in the presence of mature dendritic cells primed toward inducing a type 1 (Th-1) T cell response Such T cell optionally can be cultured on feeder cells. The mature, primed dendritic cells optionally can be irradiated prior to contacting with the T cells. Suitable culture conditions can include one or more cytokines (*e.g*., purified IL-2, Concanavalin A-stimulated spleen cell supernatant, or interleukin 15 (IL-15)) *In vitro* re-stimulation of T cells by addition of immature dendritic cells, BCG, IFNγ and the predetermined antigen can be used to promote expansion of the T cell populations

A stable antigen-specific, polarized T cell culture or T cell line can be maintained *in vitro* for long periods of time by periodic re-stimulation. The T cell culture or T cell line thus created can be stored, and if preserved (*e.g.,* by formulation with a cryopreservative and freezing) used to re-supply activated, polarized T cells at desired intervals for long term use.

In certain embodiments, activated CD8⁺ or CD4⁺ T cells can be generated according to the method of the present invention. Typically, mature, primed dendritic cells used to generate the antigen-reactive, polarized T cells are syngeneic to the subject to which they are to be administered (*e.g*., are obtained from the subject). Alternatively, dendritic cells having the same HLA haplotype as the intended recipient subject can be prepared *in vitro* using non-cancerous cells (*e.g.,* normal cells) from an HLA-matched donor. In a specific embodiment, antigen-reactive T cells, including CTL and Th-1 cells, are expanded *in vitro* as a source of cells for immunostimulation.

### In vivo Administration of Cell Populations

In another aspect of the invention, methods are provided for administration of mature, primed dendritic cells or activated, polarized T cells, or a cell population containing such cells, to a subject in need of immunostimulation. Such cell populations can include both mature, primed dendritic cell populations and/or activated, polarized T cell populations In certain embodiments, such methods are performed by obtaining dendritic cell precursors or immature dendritic cells, differentiating and maturing those cells in the presence of BCG, IFNγ and predetermined antigen to form a mature dendritic cell population primed towards Th-1 response. The immature dendritic cells can be contacted with antigen prior to or during maturation. Such mature, primed dendritic cells can be administered directly to a subject in need of immunostimulation,

In a related embodiment, the mature, primed dendritic cells can be contacted with lymphocytes from a subject to stimulate T cells within the lymphocyte population. The activated, polarized lymphocytes, optionally followed by clonal expansion in cell culture of antigen-reactive CD4⁺ and/or CD8⁺ T cells, can be administered to a subject in need of immunostimulation. In certain embodiments, activated, polarized T cells are autologous to the subject

In another embodiment, the dendritic cells, T cells, and the recipient subject have the same MHC (HLA) haplotype. Methods of determining the HLA haplotype of a subject are known in the art. In a related embodiment, the dendritic cells and/or T cells are allogenic to the recipient subject. For example, the dendritic cells can be allogenic to the T cells and the recipient, which have the same MHC (HLA) haplotype The allogenic cells are typically matched for at least one MHC allele (*e.g.*, sharing at least one but not all MHC alleles). In a less typical embodiment, the dendritic cells, T cells and the recipient subject are all allogeneic with respect to each other, but all have at least one common MHC allele in common.

According to one embodiment, the T cells are obtained from the same subject from which the immature dendritic cells were obtained, After maturation and polarization *in vitro,* the autologous T cells are administered to the subject to provoke and/or augment an existing immune response For example, T cells can be administered, by intravenous infusion, for example, at doses of about 10⁸-10⁹ cells/m² of body surface area (*see, e.g.,* Ridell et al., Science 257:238-41 (1992), incorporated herein by reference). Infusion can be repeated at desired intervals, for example, monthly. Recipients can be monitored during and after I cell infusions for any evidence of adverse effects

According to another embodiment, dendritic cells matured with BCG and IFNγ according to the present invention can be injected directly into a tumor, or other tissue containing a target antigen. Such mature cells can take up antigen and present that antigen to T cells *in vivo.*

### EXAMPLES

The following examples are provided merely as illustrative of various aspects of the invention and shall not be construed to limit the invention in any way.

### Example 1: Production of IL-10 and IL-12 Under Different Maturation Conditions:

In this example, cytokine production was determined from populations of immature dendritic cells that were contacted with the maturation agents BCG and/or IFNγ. Immature DCs were prepared by contacting peripheral blood monocytes with plastic in the presence of OptiMEM® media (Gibco-BRL) supplemented with 1% human plasma. Unbound monocytes were removed by washing. The bound monocytes were cultured in X-VIVO 15® media in the presence of 500 U GM-CSF and 500 U IL-4 per milliliter for 6 days

In a first study, immature dendritic cells were matured by addition of inactivated BCG. The cytokine production of the resulting mature dendritic cells was determined. Inactivated BCG was added at varying concentrations to immature dendritic cells in X-VIVO 15® media, followed by culturing for 24 hours at 37°C. The dilution of BCG added per milliliter is specified in the table, starting from a 4.1 x 10⁸ cfu/ml stock. Cytokine production was determined by ELISA assay using antibodies against the cytokine to be detected Briefly, an antibody specific to the cytokine (*e.g.,* IL-12 or IL-10 is used to capture the cytokine on a solid surface, The solid surface is then treated with a second, labeled antibody against the cytokine to detect the presence of the captured cytokine. The second antibody is typically labeled with enzyme to facilitate detection by colorimetric assay The results of a representative experiment are shown below in the following Table 1. The amount of cytokine, or cytokine production, is stated as pg/ml.

**Table 1**

| IL-10 and IL-12 Production by DC Matured with BCG | | | | | | | |
|---|---|---|---|---|---|---|---|
| donor | cytokine measured | No added factor | BCG 1:100 | BCG 1:250 | BCG 1:500 | BCG 1:1000 | TNFα + IL-1β |
| 2 | IL-12 p70 | <5 | 393 | 239 | 335 | <5 | <5 |
| | IL-12 p40 | nd | nd | 2852 | nd | nd | nd |
| 2 | IL-10 | 76.5 | 1206 | 700 | 338 | 153 | 380 |
| 2 | p70/IL-10 | <0.06 | 0.33 | 0.34 | 1 | <0.03 | <0.01 |
| 3 | IL-12 p70 | 249 | 318 | 260 | 74 | <5 | <5 |
| | IL-12 p40 | nd | nd | 12257 | nd | nd | nd |
| 3 | IL-10 | 305 | 426 | 162 | 124 | <5 | 70 |
| | p70/IL-10 | 0.82 | 0.75 | 1.60 | 0.60 | nd | <0.07 |

| | | | | | | | |
|---|---|---|---|---|---|---|---|
| ("nd" means not determined.) | | | | | | | |

Referring to Table 1, the results demonstrate that the addition of BCG can increase the production of cytokines IL-12, IL-10, or their subunits, although both the relative and absolute levels of cytokine production are donor-dependent. The highest levels of increase were seen for IL-12 p40 and for IL-10, suggesting that the observed low-level of IL-12 p70 (composed of p35 and p40 subunits), relative to the level of IL-12 p40, is due to a lack of IL-12 p35 production In all conditions, except one, where BCG is present, the ratio of the levels of IL-12 p70 to IL-10 is less than or equal to 1, indicating that maturation of immature dendritic cells in the presence of BCG alone is likely to polarize naive T cells towards a Th-2 response.

The effects of introducing IFNγ under similar conditions were also determined and are presented in the following Table 2. Cytokine production was measured as described above Comparing Tables 1 and 2, it is evident that addition of IFNγ in the presence of a maturation agent (*e*.*g*., BCG) increased the production of IL-12 p70. In particular, addition of IFNγ with BCG during maturation. increased IL-12 p35 production and decreased IL-10 production As a result, the ratio of IL-12 p70 to IL-10 was invariably greater than 1 upon addition of IFNγ with BCG. In some donors, and under certain conditions, the ratio of IL-12 p70 to IL-10 production can be increased to greater than 100:1 by the addition of IFNγ with BCG. Thus, these results surprisingly demonstrate that addition of IFNγ with the maturation agent BCG can dramatically increase IL-12 p70 production,

**Table 2**

| IL-10 and IL-12 Production by DC Matured with BCG and IFNγ | | | | | | | |
|---|---|---|---|---|---|---|---|
| donor | cytokine | IFNγ alone | BCG 1:100 + IFNγ | BCG 1:250 + IFNγ | BCG 1:500 + IFNγ | BCG 1:1000 + IFNγ | TNFα+I L-1β + IFNγ |
| 2 | IL-12 p70 | <5 | 1223 | 801 | 848 | 461 | 521 |
| | IL-12 p40 | <5 | nd | 23751 | 19362 | 8666 | nd |
| 2 | IL-10 | <5 | 470 | 510 | 394 | 179 | 95 |
| | p70/IL-10 | | 2.60 | 1.57 | 2.15 | 2.58 | 5.48 |
| 3 | IL-12 p70 | 212 | 6858 | 5380 | 2934 | 949 | 231 |
| | IL-12 p40 | <5 | nd | 48351 | nd | 16164 | 25645 |
| 3 | IL-10 | 141 | 254 | 241 | 157 | <5 | 163 |
| | p70/IL-10 | 1.50 | 27 | 22 | 18 | >189 | 1.41 |

| | | | | | | | |
|---|---|---|---|---|---|---|---|
| ("nd" means not determined.) | | | | | | | |

### Example 2: Downregulation of IL-10 by IFNγ is Dose-Dependent:

In this example, the ability of IFNγ in combination with BCG to downregulate IL-10 production in a population of dendritic cells is demonstrated Immature dendritic cells were prepared as described above The immature dendritic cells were incubated alone, matured in the presence of one of two concentrations of BCG (1:1000 or 1:250 dilutions of the 4.1 x 10⁸ cfu/ml stock), or exposed to IFNγ alone in concentrations ranging from 0 U to 1000 U per milliliter. IL-10 production by the resulting dendritic cells was measured by ELISA *(supra)* using a commercially available antibody (*e.g.*, from R&D Systems, Minneapolis, MN) and reported in pg/ml. In the control, immature DCs cultured alone (without addition of BCG or IFNγ) produced no detectable IL-10 In contrast, DC cultured in the presence of IFNγ alone produced a small amount of IL-10 (about 20-30 pg/ml). The amount of IL-10 produced was not dose dependent over the range of 10 U to 1000 U of IFNγ per milliliter.

In contrast, DCs produced by maturation in the presence BCG alone produced significant amounts of IL-10: about 150 pg/ml or >250 pg/ml of IL-10 in the presence of a 1:1000 or 1:250 fold dilution of the BCG stock, respectively, Addition of IFNγ to BCG during DC maturation resulted in downregulation of IL-10 production in a dose-dependent manner. For DCs cultured in the presence of the 1:1000 dilution of BCG, IL-10 production decreased from about 150 pg/ml of IL-10 (no IFNγ) to about 20-30 pg/ml of IL-10 (1000 U IFNγ). For DCs cultured in the presence of the 1:250 dilution of BCG, IL-10 production decreased from about 270 pg/ml of IL-10 (no IFNγ) to about 50 pg/ml of IL-10 (1000 U IFNγ). Thus, maturation of immature DCs in the presence of BCG and IFNγ was able to downregulate IL-10 production, and to overcome the apparent stimulation of IL-10 production induced by BCG alone.

### Example 3: Upregulation of IL-12 by IFNγ is Dose Dependent:

In this example, the ability of IFNγ to upregulate IL-12 production was demonstrated Immature dendritic cells were derived from six day monocyte cultures grown in the presence of GM-CSF and IL-4, as described above The immature DC were treated for an additional two days with various dilutions of BCG alone, or with BCG in combination with various concentrations of IFNγ. Culture supernatants were tested for the presence of IL-12 p70 by ELISA assay, as described above.

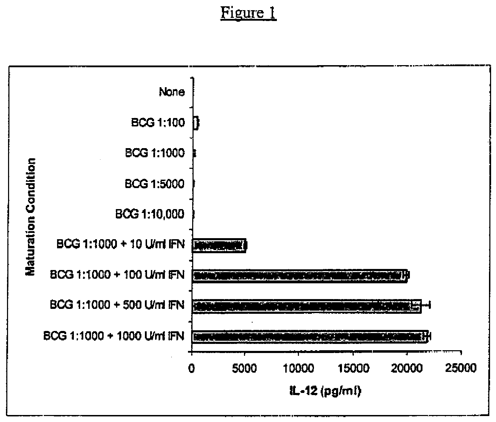

The results from a representative experiment are shown in Figure 1. For each culture, the results were determined in triplicate. In response to increasing amounts of BCG alone, a relatively low (<1000 pg/ml) mean concentration of IL-12 p70 was produced by the mature DCs. The amount of IL-12 production decreased in a dose dependent fashion as the amount of BCG increased In contrast, upon addition of IFNγ (10 U/ml) with BCG (1:1000 dilution of the stock), the amount of IL-12 increased dramatically to approximately 5000 pg/ml. Upon addition of 100U/ml of IFNy with BCG (1:1000 dilution of the stock), the amount of IFNγ increased to almost 20,000 pg/ml. Addition of IFNγ at 500 U/ml or 1000U/ml with BCG (1:1000 dilution of the stock) resulted in IL-12 levels of approximately 21,000 pg/ml and 22,000 pg/ml respectively.

In summary, although BCG alone appeared to antagonize IL-12 production, maturation of immature DCs in the presence of BCG and IFNγ dramatically increased IL-12 production.

### Example 4: Stimulation of Antigen Specific T-cells:

In this example, immature DC matured in the presence of BCG and IFNγ were shown to stimulate IFNγ production by antigen-specific T cells. A T cell line specific to influenza A was generated by incubating peripheral blood mononuclear cells (PBMC) at 2 x 10⁶ cells/ml with 5 µg/ml of influenza M1 peptide (GILGFVFTL; SEQ ID NO:3) in AIM-V® media supplemented with 5% human serum. These culture conditions result in selective expansion of those T cells specific for the influenza M1 peptide. After 2 days of culture, 20 U/ml IL-2 and 5 ng/ml IL-15 were added to the cultures After about 7 to 14 days of culture, the T cell lines were placed in cytokine-free media overnight.

The antigen-specific T cells were then co-cultured with immature DCs, with DCs matured with BCG alone, or with DCs matured with BCG and IFNγ. The ratio of antigen-specific T cells to DCs was 1:1. The T cells and DCs were incubated at 37°C for 24 hours The DCs had been either directly loaded or osmotically loaded with influenza M1 peptide. Briefly, the immature DC were harvested from culture flasks and concentrated by centrifugation. For osmotic loading, the cells were resuspended in a small volume of hyperosmotic media, followed by the addition of an equal volume of influenza M1 peptide in PBS After a ten minute incubation on ice, the cells were washed extensively. For direct loading, the cells were resuspended in an equal volume of X-VIVO 15® media and influenza M1 peptide in PBS, and incubated for 1 hour at 37°C. The cells were incubated for 2 hours at 37°C to allow for antigen processing.

After co-culture of the T cells and DCs, the T cell response was measured by ELISA quantitation of IFNγ from a 100 µl sample of culture supernatant. The results indicate that, irrespective of the method of DC loading, DCs stimulated with BCG and IFNγ are superior stimulators of antigen specific T cells. T cells co-cultured with immature DC produced very little IFNγ (<2,000 pg/ml), while T cells co-cultured with DCs matured using BCG alone were intermediate producers of IFNγ (>5,000 pg/ml) T cells co-cultured with DCs matured using BCG and IFNγ produced high levels of IFNγ (>20,000 pg/ml for osmotically-loaded DCs or >25,000 pg/ml for DCs loaded directly.)

Thus, DC matured with BCG and IFNγ were better stimulators of antigen-specific T cells, independent of the method of loading the DC with antigen.

### Example 5: De Novo Generation of Antigen Specific T Cell Responses in vitro:

T cell lines specific to keyhole limpet hemocyanin (KLH) were generated by stimulating PBMC with DC matured using either BCG, or BCG and IFNγ, and loaded with KLH or control proteins at a 10:1 T cell to DC ratio The T cells and matured DC were provided fresh media (AIM-V® media supplemented with 5% human AB serum, 20 U/ml IL-2, and 5 ng/ml IL-15) every 3 to 4 days The cells were expanded to larger flasks, as necessary. Because the overall precursor frequency to a certain antigen was low, and because naïve cells require potent stimulation to respond, stimulation was repeated 3 to 4 times in intervals of 10 to 21 days. Cells were allowed to recover overnight in cytokine-free media before re-stimulation.

A standard 3-day thymidine incorporation assay was employed to test stimulated T cell lines for KLH-specific cell proliferation. Stimulated T cells were incubated in varying DC to T cell ratios with KLH-pulsed immature dendritic cells. T cell proliferation was measured as counts per minute (CPM). Cellular proliferation, or production of cytokines, in response to stimulation were taken as evidence of antigen-specific response. To control for antigen specificity, a negative control antigen (influenza A virus) was also included in the assay

When DCs matured by BCG alone were used to stimulate T cells, T cell proliferation was consistently low (<5,000 CPM) This low level of proliferation was a low whether the DCs were contacted with KLH or influenza A virus. A low level of proliferation was also observed in response to incubation with immature DCs. No significant difference was observed between the three groups of DCs used at responder to stimulator ratios of 50:1, 25:1, or 12.5:1 (T cells to DCs), In contrast, dendritic cells matured with BCG and IFNγ were used to stimulate the 1 cells, KLH-pulsed DCs induced consistently higher T cell proliferation (approximately 10,000-33,000 CPM) than did immature DCs or mature DCs pulsed with influenza A. For mature DCs pulsed with KLH antigen, T cell proliferation increased in proportion to an increase in the responder to stimulator ratio.

T cell effector function was also monitored by cytokine secretion. The KLH specific T cells lines (generated as described above) were stimulated using DC matured with either BCG alone or with BCG and IFNγ. The stimulated T cell lines were tested for cytokine production by intracellular cytokine staining after the cells were non-specifically stimulated with anti-CD3 antibody (50 ng/ml) and PMA (5 ng/ml). Cytokine production was measured as a percentage of cells producing a particular cytokine A very low to undetectable percentage (<< 5%) of sampled cells produced intracellular IL-2, IL-4, IL-5, or IL-10 IL-5 and IL-10 were not detected in T cells stimulated by DCs matured with BCG and IFNγ. T Cells stimulated by DCs matured by BCG alone produced low levels of IFNγ (<10%) and TNF-α (<15%). In contrast, significant proportions of T cells stimulated with DCs matured with IFNγ and BCG produced IFNγ (approximately 35%) and TNFα (>45%). IFNγ is a known stimulator of IL-12 production. Thus, by stimulating T cells with DCs matured with BCG and IFNγ, the T cells are polarized towards a type-1 (Th-1) response,

### Example 6: Induction of the Th-1 Cytokine Tumor Necrosis Factor α (TNFα):

In this example the ability of the combination of BCG and IFNγ to upregulate the type-1 cytokine tumor necrosis factor α (TNFα) was demonstrated Briefly, Immature dendritic cells were derived as described above and prown in the presence of GM-CSF and IL-4 The immature DCs were cultured with either BCG alone or in combination with IFNγ for about 24 h. Subsequently, a protein transport inhibitor (GolgiPlug™, PharMingen) was added to block the transport of the produced cytokines from the golgi complex, and the cells were incubated overnight The cells were then harvested, permeabilized and stained internal with a fluorescently label antibody specific for TNFα or an isotype control antibody using methods well known in the art. The frequency of DCs positive for TNFα and the fluorescence intensity of the cells were determined by FACS analysis (Table 3). Maturation of the DCs with BCG in the presence of IFNγ was found to enhance the capacity of the DCs to produce the Th-1 cytokine TNFα.

**Table 3**

| TNFα Production by DCs Matured in the Presence of BCG With or Without INFγ | | |
|---|---|---|
| Maturation Conditions | % Positive Cells | Mean Fluoresence Intensity |
| BCG | 3.9 | 99 |
| BCG + IFNγ | 31.5 | 192 |

### Example 7: Induction of Response Against Cell-Associated Antigen:

In this example DCs matured in the presence or BCG and IFNγ where demonstrated to elicit a significantly higher tumor-specific T cell INFγ release and similar levels of antigen-specific cytotoxicity as compared to DCs matured with BCG alone. Immature dendritic cells were isolated as set forth above and cultured in the presence of GM-CSF and IL-4. The DCs were then loaded with either whole tumor cells (A549) previously infected with recombinant adenovirus expressing either green fluorescent protein (GFP) or the M1 protein of Influenze A virus. The DCs were matured 24 h later with either BCG or BCG in combination with IFNγ. The tumor loaded DCs or GFP or M1-expression tumor cells were used to stimulate an autologous M1-specific T cell line. Twenty-four h later, cell culture supernatants were harvested and run on a standard IFNγ ELISA. Only DCs loaded with M1-expression tumor cells were able to stimulate IFNγ release and DCs matured in BCG plus IFNγ were significantly more potent at inducing this response than either immature or BCG matured DCs.

**Table 4**

| Induction of Response Against Cell-Associated Antigens | |
|---|---|
| Maturation Conditions | IFNγ Release |
| None | 10,379 |
| BCG | 15,114 |
| BCG + IFγN | 75,546 |

The previous examples are provided to illustrate, but not to limit, the scope of the claimed inventions. Other variants of the inventions will be readily apparent to those of ordinary skill in the art and encompassed by the appended claims. All publications, patents, patent applications and other references cited herein and are also incorporated by reference herein in their entirety.

The invention will now be defined by the following clauses:
1. A method for producing a mature dendritic cell population, comprising:
   providing immature dendritic cells; and
   contacting the immature dendritic cells with an effective amount of BCG and Interferon gamma (IFNγ) under culture conditions suitable for maturation of the immature dendritic cells to form a mature dendritic cell population;
   wherein the mature dendritic cell population produces an increased ratio of Interleukin 12 to Interleukin 10 than an immature dendritic cell population not contacted with BCG and IFNγ during maturation.
2. The method of clause 1, further comprising contacting the immature dendritic cells with a predetermined antigen prior to contacting with BCG and IFNγ
3. The method of clause 1, further comprising simultaneously contacting the immature dendritic cells with a predetermined antigen, BCG and IFNγ.
4. The method of clause 2 or 3, wherein the predetermined antigen is a tumor specific antigen, a tumor associated antigen, a viral antigen, a bacterial antigen, tumor cells, bacterial cells, a cell lysate, a membrane preparation, a recombinantly produced antigen, a peptide antigen, or an isolated antigen
5. The method of clause 1, further comprising:
   isolating monocytic dendritic cell precursors; and
   culturing the precursors in the presence of a differentiating agent to form the immature dendritic cells.
6. The method of clause 5, wherein the differentiating agent is GM-CSF, Interleukin 4, a combination of GM-CSF and Interleukin 4, or Interleukin 13
7 The method of clause 5, wherein the monocytic dendritic cell precursors are isolated from a human subject
8. The method of clause 1, wherein the mature dendritic cells produce a ratio of IL-12 to IL-10 of at least about 1:1, at least about 10:1, at least about 100:1
9 A method for producing a mature dendritic cell population, comprising:
   providing immature dendritic cells; and
   contacting the immature dendritic cells with an effective amount of BCG and Interferon gamma (IFNγ) under culture conditions suitable for maturation of the immature dendritic cells to form a mature dendritic cell population;
   wherein the mature dendritic cell population produces a type 1 immune response
10 The method of clause 9, further comprising contacting the immature dendritic cells with a predetermined antigen prior to contacting with BCG and IFNγ.
11. The method of clause 9, further comprising simultaneously contacting the immature dendritic cells with a predetermined antigen, BCG and IFNγ,
12 The method of clause 10 or 11, wherein the predetermined antigen is a tumor specific antigen, a tumor associated antigen, a viral antigen, a bacterial antigen, tumor cells, bacterial cells, recombinant cells expressing an antigen, a cell lysate, a membrane preparation, a recombinantly produced antigen, a peptide antigen, or an isolated antigen.
13. The method of clause 9, further comprising:
   isolating monocytic dendritic cell precursors; and
   culturing the precursors in the presence of a differentiating agent to form the immature dendritic cells.
14. The method of clause 13, wherein the differentiating agent is GM-CSF, Interleukin 4, a combination of GM-CSF and Interleukin 4, or Interleukin 13.
15. The method of clause 9, wherein the monocytic dendritic cell precursors are isolated from a human subject.
16. The method of clause 9, wherein the mature dendritic cells produce a ratio of IL-12 to IL-10 of at least about 1:1, at least about 10:1, or at least about 100:1.
17 A composition for activating T cells, comprising:
   a dendritic cell populations matured with an effective concentration of BCG and IFNγ under suitable conditions for maturation; and
   a predetermined antigen;
   wherein the dendritic cell population produces an increased ratio of Interleukin 12 (IL-12) to Interleukin 10 (IL-10) than a mature dendritic cell population contacted with BCG without IFNγ during maturation,
18. The composition of clause 17, wherein the dendritic cell population produces IL-12 to IL-10 in a ratio of at least about 10:1
19. The composition of clause 17, wherein the dendritic cell population produces IL-12 to IL-10 in a ratio of at least about 100:1.
20. An isolated, immature dendritic cell population, comprising:
   isolated immature monocytic dendritic cells, and an effective concentration of BCG and IFNγ to induce maturation of the immature dendritic cells;
   wherein the resulting mature dendritic cells produce more Interleukin 12 (IL-12) to Interleukin 10 (IL-10).
21. The method of clause 20, further comprising a predetermined antigen.
22. The cell population of clause 20, further comprising isolated T cells
23. The cell population of clause 22, wherein the T cells are naive T cells
24. The composition of clause 20, further comprising isolated lymphocytes.
25. A method for producing T cells, comprising:
   providing immature dendritic cells;
   contacting the immature dendritic cells with a predetermined antigen;
   contacting the immature dendritic cells with an effective concentration of BCG and IFNγ under culture conditions suitable for maturation of the immature dendritic cells to form mature dendritic cells; and
   contacting the mature dendritic cells with naïve T cells to form an activated T cells producing IFNγ.
26. The method of clause 25, wherein the predetermined antigen is a tumor specific antigen, a tumor associated antigen, a viral antigen, a bacterial antigen, tumor cells, bacterial cells, recombinant cells expressing an antigen, a cell lysate, a membrane preparation, a recombinantly produced antigen, a peptide antigen, or an isolated antigen.
27. The method of clause 25, wherein the immature dendritic cells are contacted simultaneously with the predetermined antigen, BCG and IFNγ,
28. The method of clause 25, further comprising:
   isolating monocytic dendritic cell precursors; and
   culturing the precursors in the presence of a differentiating agent to form the immature dendritic cells.
29 The method of clause 28, wherein the differentiating agent is GM-CSF, Interleukin 4, a combination of GM-CSF and Interleukin 4, or Interleukin 13.
30. The method of clause 28, wherein the monocytic dendritic cell precursors are isolated from a human subject.
31. The method of clause 25, wherein the immature dendritic cells and T cells are autologous to each other.
32 Isolated mature dendritic cells producing more Interleukin 12 (IL-12) to Interleukin 10 (IL-10) prepared by maturation of immature dendritic cells with a composition comprising effective concentrations of BCG and IFNγ under conditions suitable for the maturation of the dendritic cells
33. The isolated, mature dendritic cells of clause 32, further comprising a predetermined antigen,
34. Isolated mature dendritic cells loaded with a predetermined antigen, the dendritic cells producing more Interleukin 12 (IL-12) than Interleukin 10 (IL-10).
35. The isolated mature dendritic cells of clause 34, wherein the cells produce at least 10-fold more IL-12 than IL-10.
36. A method for producing a type 1 immune response in an animal, comprising:
   providing immature dendritic cells;
   contacting the immature dendritic cells with effective amounts of BCG and Interferon gamma (IFNy) and a predetermined antigen under culture conditions suitable for maturation of the immature dendritic cells to form mature dendritic cells;
   contacting the mature dendritic cells with naïve T cells to form activated T cells producing more Interleukin 12 (IL-12) to Interleukin 10 (IL-10); and
   administering the activated T cells to the animal.
37. The method of clause 36, wherein the predetermined antigen is a tumor specific antigen, a tumor associated antigen, a viral antigen, a bacterial antigen, tumor cells, bacterial cells, recombinant cells expressing an antigen, a cell lysate, a membrane preparation, a recombinantly produced antigen, a peptide antigen or an isolated antigen.
38. The method of clause 36, wherein the immature dendritic cells are simultaneously contacted with the predetermined antigen, BCG and IFNγ.
39. The method of clause 36, wherein the immature dendritic cells are contacted with the predetermined antigen prior to contacting with BCG and IFNγ
40. The method of clause 36, further comprising:
   isolating monocytic dendritic cell precursors from the animal; and
   culturing the precursors in the presence of a differentiating agent to form the immature dendritic cells.
41. The method of clause 40, wherein the differentiating agent is GM-CSF, Interleukin 4, a combination of GM-CSF and Interleukin 4, or Interleukin 13.
42. The method of clause 36, wherein the immature dendritic cells and T cells are autologous to the animal.
43. The method of clause 36, wherein the immature dendritic cells and T cells are allogenic to the animal
44. The method of clause 36, wherein the immature dendritic cells and T cells have the same MHC haplotype as the animal.
45 The method of clause 36, wherein the animal is human.

## Claims

1. An *ex vivo* or *in vitro* method for producing a mature dendritic cell population specific for a predetermined antigen and producing an increased ratio of Interleukin 12 (IL-12) to Interleukin 10 (IL-10), wherein the increased ratio of IL-12 to IL-10 is increased compared to an immature dendritic cell population no contacted with Bacillus-Calmette-Guerin (BCG) and Interferon γ (IFNγ) during maturation, comprising:
contacting a provided cell population enhanced for immature dendritic cells with an effective amount of BCG and IFNγ under culture conditions suitable for maturation of the immature dendritic cells to form the mature dendritic cell population; and
contacting the provided cell population enhanced for immature dendritic cells with the predetermined antigen.

2. An *ex vivo* or *in vitro* method for producing a mature dendritic cell population, wherein the mature dendritic cell population produces a type 1 immune response specific for a predetermined antigen, comprising:
contacting a cell population enhanced for immature dendritic cells with an effective amount of BCG and IFNγ under culture conditions suitable for maturation of the immature dendritic cells to form the mature dendritic cell population; and
contacting the cell population enhanced from immature dendritic cells with a predetermined antigen.

3. The method of any one of claims 1 and to 2, wherein the mature dendritic cells produce a ratio of IL-12 to IL-10 of at least about 1:1, at least about 10:1, or at least about 100:1.

4. The method of any one of claims 1 to 3, further comprising:
culturing isolated monocytic dendritic cell precursors in the presence of a monocyte dendritic cell differentiating agent to form the immature dendritic cells.

5. The method of claim 4, wherein the monocyte differentiating agent is GM-CSF, or GM-CSF in combination with Interleukin 4 (IL-4), or Interleukin 13 (IL-13).

6. The method of claim 4, wherein the monocytic dendritic cell precursors are human.

7. The method of any one of claims 1 to 6, wherein the predetermined antigen is a tumor specific antigen, a tumor associated antigen, a viral antigen, a bacterial antigen, a tumor cell, a bacterial cell, a recombinant cell expressing an antigen, a cell lysate, a membrane preparation, a recombinantly produced antigen, a peptide antigen, or an isolated antigen.

8. The method of any one of claims 1 to 7, wherein the immature dendritic cells are contacted simultaneously with the predetermined antigen, BCG and IFNγ, or are contacted with the predetermined antigen prior to contacting with BCG and IFNγ.

9. Use of the mature dendritic cells produced by any one of claims 1 to 8 for treating cancer, a viral infection, or a bacterial infection.

10. Use of the mature dendritic cell population produced by any one of claims 1 to 8 for inducing a type 1 immune response against a tumor specific antigen a tumor associated antigen, a viral antigen, a bacterial antigen, a tumor cell, a bacterial cell, a recombinant cell expressing an antigen, a cell lysate, a cell membrane preparation, a recombinantly produced antigen, a peptide antigen, or an isolated antigen.

11. Use of the mature dendritic cell population produced by any one of claims 1-8 in re-stimulating T cells *in vitro.*

12. A composition for use in inducing a type 1 immune response in a subject, wherein the composition comprises the mature dendritic cell population produced by the methods of any one of claims 1-8.

13. A composition for use in inducing a type 1 immune response in a subject, wherein the composition comprises mature dendritic cell population produced by contacting provided immature dendritic cells with an effective amount of Bacillus-Calmette-Guerin (BCG) consisting of 10⁵ to 10⁷ cfu per ml of tissue culture media and 100 to 1000 Units of Interferon gamma (IFNγ) per ml of tissue culture media under culture conditions suitable for maturation of the immature dendritic cells to form the mature dendritic cell population.

14. A composition for use in inducing a type 1 immune response in a subject, wherein the composition comprises activated T cells produced by contacting provided immature dendritic cells with an amount of Bacillus-Calmette-Guerin (BCG) consisting of 10⁵ to 10⁷ cfu per ml of tissue culture media and 100 to 1000 Units of Interferon gamma (IFNγ) per ml of tissue culture media and a predetermined antigen under culture conditions suitable for maturation of the immature dendritic cells to form a mature dendritic cell population or mature dendritic cells; wherein the mature dendritic cell population produces at least 10-fold more Interleukin 12 than Interleukin 10 than a similar dendritic cell population cultured in the presence of BCG without IFNγ, and contacting the mature dendritic cells with naive T cells to form activated T cells producing IFNγ.


SEQUENCE LISTING

<110> NORTHWEST BIOTHERAPEUTICS, INC.
      BOSCH, Marnix L.

<120> COMPOSITIONS AND METHODS FOR PRIMING MONOCYTIC
      DENDRITIC CELLS AND T CELLS FOR THE TH-1 RESPONSE

<130> 020093-29-1PC

<140> PCT/US02/28620
<141> 2002-09-06

<150> 60/317,569
<151> 2001-09-06

<160> 3

<170> PatentIn Ver. 2.1

<210> 1
<211> 9
<212> PRT
<213> Homo sapiens

<400> 1
Leu Leu His Gln Thr Asp Ser Ala Val
  1               5

<210> 2
<211> 9
<212> PRT
<213> Homo sapiens

<400> 2
Ala Leu Phe Asp Ile Glu Ser Lys Val
  1               5

<210> 3
<211> 9
<212> PRT
<213> Influenza A virus

<400> 3
Gly Ile Leu Gly Phe Val Phe Thr Leu
  1               5